(19) Europäisches Patentamt
European Patent Office
Office européen des brevets

(11) Veröffentlichungsnummer: **0 156 310**
**B1**

(12) # EUROPÄISCHE PATENTSCHRIFT

(45) Veröffentlichungstag der Patentschrift: **28.10.87**

(51) Int. Cl.⁴: **C 07 C 57/13**, C 07 C 51/567 // C23F11/12, C10M129/26

(21) Anmeldenummer: **85103306.8**

(22) Anmeldetag: **21.03.85**

(54) **Verfahren zur Umsetzung von Olefinen mit Maleinsäureanhydrid und verwendung der erhaltenen Bernsteinsäureanhydride zur Herstellung von Korrosionsschutzmitteln und Mineralölhilfsmitteln.**

(30) Priorität: **29.03.84 DE 3411531**

(43) Veröffentlichungstag der Anmeldung: **02.10.85 Patentblatt 85/40**

(45) Bekanntmachung des Hinweises auf die Patenterteilung: **28.10.87 Patentblatt 87/44**

(84) Benannte Vertragsstaaten: **BE DE FR GB IT NL**

(56) Entgegenhaltungen: **US - A - 2 768 175**

(73) Patentinhaber: **BASF Aktiengesellschaft, Carl-Bosch-Strasse 38, D-6700 Ludwigshafen (DE)**

(72) Erfinder: **Bronstert, Klaus, Dr., Gartenstrasse 26, D-6719 Carlsberg (DE)**
Erfinder: **Vogel, Hans-Henning, Dr., Hans-Purrmann-Strasse 7c, D-6710 Frankenthal (DE)**
Erfinder: **Rath, Hans Peter, Dr., Friedhofstrasse 7, D-6718 Gruenstadt (DE)**

**EP 0 156 310 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Umsetzung von olefinisch ungesättigten Kohlenwasserstoffen, insbesondere von dimeren bis polymeren Isobutenderivaten, die ein mittleres Molekulargewicht mit einem Zahlenmittel $M_n$ von 100 bis 3000 aufweisen, mit Maleinsäureanhydrid im molaren Mengenverhältnis Maleinsäureanhydrid zu Olefin von 0,2 bis 3,0 in Gegenwart von 1 bis 5000 Gew.-ppm, bezogen auf das Olefin, eines Nebenreaktionen verhindernden Zusatzstoffes bei Temperaturen zwischen 150 und 280°C unter Bildung der entsprechenden Bernsteinsäureanhydride sowie deren Verwendung zur Herstellung von Korrosionsschutzmitteln oder Mineralölhilfsmitteln.

Bei Umsetzungen von Olefinen mit Maleinsäureanhydrid entstehen bei erhöhten Temperaturen die entsprechenden Addukte z.B. nach dem folgenden Reaktionsschema:

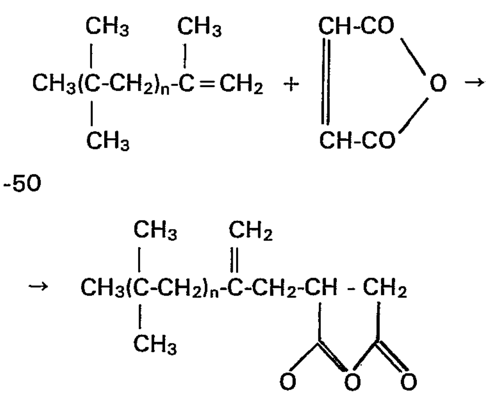

n = 1-50

die Polyisobutylen-Bernsteinsäureanhydride. Für derartige Umsetzungen in der Wärme werden aber sehr lange Reaktionszeiten benötigt, beispielsweise 18 Stunden bei einer Temperature von 230°C (vgl. US-A-3 306 907), so dass eine Beschleunigung der Additionsreaktion erstrebenswert ist.

Es ist bereits bekannt, zur Erfüllung dieses Erfordernisses die Umsetzung von olefinisch ungesättigten Kohlenwasserstoffen mit Maleinsäureanhydrid in Gegenwart von katalytischen Mengen eines Zusatzstoffes vorzunehmen. So werden nach bekannten Verfahren die Reaktionszeiten auf ein zufriedenstellendes Mass dadurch gesenkt, dass man die Additionsreaktionen in Anwesenheit geringer Mengen, im allgemeinen in Gegenwart von 1 bis 5000 Gew.-ppm, von Substanzen wie Furanderivate (vgl. US-A-4 388 471), Jod (vgl. GB-A-1 356 802), Brom (vgl. GB-A-1 480 453), eines α-Bromdialkylketons (vgl. US-A-3 953 475 und 3 954 812), Chlorwasserstoff oder Calciumbromid (vgl. US-A-3 935 249), eines Hydantoinderivates (vgl. US-A-3 927 041), p-Toluolsulfonsäure (vgl. US-A-3 855 251), eines Nickelsalzes (vgl. GB-A-2 081 274) oder eines Bromphenols (vgl. US-A-4 278 604) ausführt.

Bei diesen bekannten Verfahren wird aber häufig das Olefin nur zur einem geringen Grade umgewandelt. Zudem besteht bei der Verwendung von Halogenverbindungen auch noch ein erhebliches Risiko wegen der Giftigkeit der entstandenen Reaktionsmischung und der Zersetzung von Polyisobutylen bei höheren Temperaturen. Die bekannten Verfahren weisen auch noch den Nachteil auf, dass sich während der Umsetzung Feststoffe von brauner bis schwarzer Farbe bilden, die die Kesselwandungen oder in schlimmeren Fällen das Reaktionsprodukt verschmutzen. Noch ungünstiger ist die Bildung harzartiger Rückstände, die das Reaktionsprodukt unbrauchbar machen, wenn es aufgrund des Molekulargewichtes nicht mehr destillativ gereinigt werden kann.

Der Erfindung liegt die Aufgabe zugrunde, die oben erwähnten Nachteile zu vermeiden und ein Verfahren zur adduktbildenden Reaktion von Olefinen mit Maleinsäureanhydrid aufzufinden, bei dem der Umsatz beschleunigt, die Ausbeute erhöht und die Harzbildung des Maleinsäureanhydrids vermindert wird.

Diese Aufgabe wird erfindungsgemäss dadurch gelöst, dass in dem eingangs beschriebenen Verfahren als die Nebenreaktionen verhindernde Zusatzstoffe Alkoxide des Titans, Zirkons, Vanadins oder Aluminiums verwendet werden. Bevorzugt werden $C_2$- bis $C_4$-Alkoxide eingesetzt.

Als olefinisch ungesättigte Kohlenwasserstoffe kommen alle Verbindungen und deren Gemische in Frage, die endständige oder innenliegende Doppelbindungen aufweisen und ein mittleres Molekulargewicht ($M_n$) zwischen 100 und 3000 besitzen.

Unter olefinisch ungesättigten Kohlenwasserstoffen werden insbesondere monomere, oligomere oder polymere $C_8$- bis $C_{14}$-Alkene verstanden, deren Ketten gegebenenfalls verzweigt sind und die ein mittleres Molekulargewicht (Zahlenmittel) $M_n$ von 100 bis 3000 aufweisen, wobei das mittlere Molekulargewicht $M_n$ mit Hilfe des osmotischen Drucks der Chloroformlösung bestimmt werden kann. Zu den olefinisch ungesättigten Kohlenwasserstoffen, die der Additionsreaktion unterworfen werden, gehören beispielsweise 1-Octen, 2-Methyl-penten, 2-Methyl-5-propyl-1-hexen, 3-Cyclohexyl-1-buten oder die Oligomeren von $C_2$- bis $C_{20}$-Olefinen, beispielsweise die Oligomeren von Ethylen, Propylen, 1-Buten, Isobuten, 1-Hexen, 1-Octen usw. sowie die Polyisobutene mit $M_n$ von 250 bis 3000 und das Diisobuten. Bevorzugte olefinisch ungesättigte Kohlenwasserstoffe sind dimere bis polymere Isobutenderivate, also das Diisobuten, oligomere Isobutene mit $M_n$ zwischen 200 und 350 und polymere Isobutene mit $M_n$ zwischen 250 und 3000. Zur Herstellung der oligomeren und polymeren Isobutene wird auf die einschlägige Literatur verwiesen (vgl. z.B. H. Güterbock, «Chemiche Technologie der Kunststoffe, Polyisobutylen», Springer-Verlag, Berlin, Göttingen, Heidelberg 1959 oder «Ullmanns Encyklopädie der technischen Chemie», 4. Auflage, Band 19, 1980, Seiten 211 bis 223, Verlag Chemie GmbH, D-6940 Weinheim).

Die Umsetzung der olefinisch ungesättigten Kohlenwasserstoffe mit Maleinsäureanhydrid erfolgt im molaren Mengenverhältnis, d.h. im Stoffmengenverhältnis, bezogen auf Mole der Komponenten, Maleinsäureanhydrid zu Olefin von 0,2 bis 3,0, bevorzugt 0,5 zu 2,0. Insbesondere bevorzugt ist ein Verfahren, bei dem äquimolare Mengen Olefin und Maleinsäureanhydrid eingesetzt werden können.

Zur Vermeidung von Nebenreaktionen bei der Additionsreaktion des Maleinsäureanhydrids arbeitet man in Gegenwart von 1 bis 5000, bevorzugt 5 bis 1000 Gew.-ppm, bezogen auf das eingesetzte Olefin, eines Zusatzstoffes, der die gewünschte Additionsreaktion beschleunigen soll. Derartige Zusatzstoffe sind, wie eingangs erwähnt, bekannt. Sie bewirken eine Verringerung der Reaktionszeit und eine Erhöhung des Umsetzungsgrades des jeweiligen Olefins. Als haupsächliche Nebenreaktion ist zu erwähnen die Bildung von Polymaleinsäureanhydrid, das als fester Rückstand anfällt, oder Polymaleinsäureanhydrid mit olefinischem Anteil aus radikalischer Copolymerisation von Olefin und Maleinsäureanhydrid, das meist in Form eines klebrigen Harzes anfällt.

Die Additionsreaktion unter Bildung der entsprechenden Bernsteinsäureanhydride erfolgt in der Wärme bei Temperaturen zwischen 150 und 280, bevorzugt 180 und 230°C. Die Umsetzung selbst kann in einem Rührautoklaven vorgenommen werden, ein Lösungsmittel ist nicht erforderlich. Die Reaktionszeiten liegen gewöhnlich bei bis zu 20 Stunden. Zweckmässigerweise wird in Anwesenheit eines Inertgases so verfahren, dass im Reaktionsgefäss zu Beginn der Reaktion ein Druck zwischen 5 und 1000, bevorzugt 5 und 100 mbar eingestellt wird. Je nach Olefinkomponente herrscht nach dem Hochheizen auf Reaktionstemperatur in der Reaktionszone ein Druck von 1 bis 10 bar. Als Inertatmosphäre wird bevorzugt Stickstoff- oder Kohlendioxidatmosphäre verwendet. Nachdem die Reaktion beendet ist, lässt man abkühlen und arbeitet bevorzugt die Reaktionsmasse destillativ auf. Die Reaktionsteilnehmer sollen nach Möglichkeit wasserfrei sein.

Nach erfindungsgemässem Verfahren sollen als Zusatzstoffe Alkoxide, bevorzugt die $C_2$- bis $C_4$-Alkoxide, des Titans, Zirkons, Vanadins oder Aluminiums eingesetzt werden. Derartige Verbindungen sind an sich bekannt und beispielsweise im Alfa-Catalog 1981 der Firma Ventrum GmbH, Zeppelinstrasse 7, D-7500 Karlsruhe 1 als sog. Alfa-Produkte aufgeführt. Insbesondere geeignete Alkoxide sind die Verbindungen: Titan(IV)butoxid = $Ti(C_4H_9O)_4$, Titan(IV)i-butoxid = $Ti[(CH_3)_2CHCH_2O]_4$, Titan(IV)ethoxid = $Ti(C_2H_5O)_4$, Titan(IV)i-propoxid = $Ti(OC_3H_7)_4$, Titan(IV)n-propoxid = $Ti(C_3H_7O)_4$, Zirkon n-butoxid-Butanolkomplex = $(C_4H_9O)_4$-Zr · $C_4H_9OH$, Zirkon-i-propoxid = $Zr(OC_3H_7)$ · $C_3H_7OH$, Zirkon-n-propoxid = $Zr(OC_3H_7)_4$, Vanadin(V)tri-n-butoxid-oxid = $VO(OC_4H_9)_3$, Vanadin(V)triethoxid-oxid = $VO(OC_2H_5)_3$, Vanadin(V)tri-i-propoxidoxid = $VO(OC_3H_7)_3$, Vanadin(V)tris-n-propoxid-oxid = $VO(OC_3H_7)_3$, Aluminium-i-butoxid = $Al(OC_4H_9)_3$, Aluminium-n-butoxid = $Al(OC_4H_9)_3$, Aluminium-s-butoxid = $Al(OC_4H_9)_3$, Aluminium-t--butoxid = $Al(OC_4H_9)_3$ oder Aluminium-i-propoxid = $Al(OC_3H_7)_3$. Die erwähnten Alkoxide liegen in flüssigem Zustand, gegebenenfalls als Komplexverbindung mit dem entsprechenden Alkohol, vor und werden in dieser Form bei der Additionsreaktion verwendet. Sie werden mit einem Reinheitsgrad von 95 bis 99 Gew.-%, bei den Alkoxiden des Aluminiums von 90 bis 99 Gew.-%, eingesetzt. Die zu verwendenden Alkoxide sind in der Reaktionsmischung löslich.

Die mit der Erfindung erzielbaren Vorteile sind insbesondere darin zu sehen, dass die Umsetzung der olefinisch ungesättigten Kohlenwasserstoffe mit Maleinsäure auf eine Reaktionszeit zwischen 3,5 bis 7,0, bevorzugt 3,5 bis 5,0 Stunden, beschleunigt und das Olefin bei der Reaktion isomerisiert wird, wodurch eine Ausbeuteerhöhung erreicht wird. Gleichzeitig wird eine Minderung der Harzbildung oder Isomerisierung zu Fumarsäure aus Maleinsäureanhydrid beobachtet. Ein weiterer Vorteil ist die Abwesentheit von Lösungsmitteln und die Tatsache, dass keine halogenhaltigen giftigen Reaktionsprodukte entstehen.

Die erhaltenen maleinisierten Olefinreaktionsprodukte mit mittleren Molekulargewichten ($M_n$) von 200 bis 350 werden zur Herstellung von Korrosionsschutzmitteln für wässrige oder organische Systeme verwendet. Die erhaltenen Olefinbernsteinsäureanhydride mit mittleren Molekulargewichten ($M_n$) von 250 bis 3000 können auf einfache Weise in Verbindungen umgewandelt werden, die sich als Mineralölhilfsmittel wie Schmiermittelzusätze eignen. Hierzu können sie mit mehrwertigen Alkoholen in die entsprechenden Ester oder mit Aminen in die entsprechenden Salze, Amide oder Imide überführt werden, welche dann, z.B. in Schmieröle, eingemischt werden (vgl. GB-A-1 483 729 und GB-A-2 081 274). Derartige Verfahren sind bekannt, so dass sie hier nicht weiter erläutert werden müssen.

*Beispiel 1*

500 g Polyisobuten ($M_n$ 930), 50 g Maleinsäureanhydrid und 500 ppm Titan(IV)n-butoxid [$Ti(C_4H_9O)_4$] werden in einem 1 l Autoklav aus V2A-Stahl, der mit Magnetstäbchen gerührt wird, vorgelegt, mit Stickstoff gespült, auf 2 mbar evakuiert und dann im Ölbad auf 225°C unter starkem Rühren aufgeheizt. Nach 4 h bei 225°C wird der Reaktor entspannt und nicht umgesetztes Maleinsäureanhydrid (MSA) destillativ bei 2 mbar Vakuum abgetrennt.

Das verbleibende Reaktionsgemisch enthält 40 Gew.-% nichtumgesetztes Polyisobuten (PIB) und 60 Gew.-% Polyisobutenylbernsteinsäureanhydrid (PIBSA). Die Analyse erfolgt durch präparative, säulenchromatographische Trennung mit Kieselgel als Adsorbens, Hexan als Elutionsmittel für PIB und Toluol/Aceton im Gewichtsverhältnis 9:1 für PIBSA. Der endständige Doppelbindungsanteil des unumgesetzten PIB beträgt 14%. Ein Rückstand in fester oder harzartiger Form wird nicht im Produkt gefunden. Lediglich an Rührer und Wandungen finden sich Ablagerungen, die nach Entfernung von PIB und PIBSA durch eine Toluolwäsche in Aceton gelöst werden. Die Lösung wird in einen Kolben überführt, eingedampft und der Rückstand gravimetrisch bestimmt. Er beträgt weniger als 0,2 g.

*Vergleichsbeispiel 1*

Bei völlig analoger Arbeitsweise gemäss Beispiel 1 ohne Titan(IV)n-butoxid wird ein Umsatz von nur 50 Gew.-%, bezogen auf PIB, ein endständiger Doppelbindungsgehalt des unumgesetzten PIB von 14% und ein Rückstand von 2,6 g gefunden. Darin ist auch fester Rückstand enthalten, der durch Druckfiltration des Ansatzes, Toluolwäsche des Filterrückstandes und anschliessender Trocknung erhalten wurde.

*Beispiel 2*

Ein technisches Gemisch aus 2,4,4-Trimethylpenten(1) und -(2) wird mit 500 ppm Titan(IV)n-butoxid [$Ti(C_4H_9O)_4$] in einem 1 l V2A-Autoklav, der mit Magnetstäbchen gerührt wird, vorgelegt, mit Stickstoff gespült, auf 15 mbar evakuiert und dann im Ölbad auf 225°C aufgeheizt. Die Zugabe von 350 g MSA erfolgt mit einer Geschwindigkeit von 100 g/h und einer Startmenge von 50 g mittels Dosierpumpe. Nach 3 h ist diese Zugabe beendet und man lässt den Ansatz 4 h nachreagieren. Nach dem Abkühlen wird der Ansatz filtriert und der Rückstand mit Toluol sorgfältig gewaschen und getrocknet.

Auch das Reaktionsgefäss wird mit Toluol gespült und der verbleibende Belag in Aceton gelöst und im Kölbchen eingedampft. Die Summe der Rückstände aus Filtration und Eindampfung ist kleiner als 0,2 g. Der Olefinumsatz beträgt 70 Gew.-% und wird destillativ bestimmt, der MSA-Umsatz ist quantitativ. Der Anteil des Restolefins an 2,4,4-Trimethylpenten(1) ist 33 Gew.-%.

*Vergleichsbeispiel 2*

Bei völlig analoger Arbeitsweise gemäss Beispiel 2 ohne Titan(IV)n-butoxid wird ein vergleichbarer Umsatz erzielt, jedoch 0,6 g Rückstand erhalten und im Restolefin einen 2,4,4-Trimethylpenten(1)-Gehalt von nur 11%.

*Umsetzung ungesättigter Kohlenwasserstoffe mit Maleinsäureanhydrid*

| Beispiel | Olefin Art | Olefin Menge [g] | MSA [g] | Zusatz [ppm] | Druck [mbar] 20°C | Druck [mbar] 225°C | Temperatur [°C] | Dauer [h] | Umsatz* [Gew.-%] | Rückstand [g] | Doppelbindungsanteil in %** 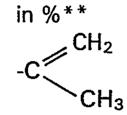 |
|---|---|---|---|---|---|---|---|---|---|---|---|
| 1 | PIb ($M_n$ 930) | 500 | 50 | 500 | 2 | 500-1200 | 225 | 4 | 57 | <0,2 | 14 |
| Vergleich | PIB ($M_n$ 930) | 500 | 50 | — | 2 | 500-1200 | 225 | 4 | 50 | 2,6 | 5 |
| 2 | 2,4,4-Trimethylpenten | 500 | 350 | 500 | 15 | 9-10 bar | 225 | 7 | 70 | 0,2 | 33 |
| Vergleich | » | 500 | 350 | — | 15 | 9-10 bar | 225 | 7 | 68 | 0,6 | 11 |

* bezogen suf Olefin

** bezogen auf die Gesamtheit der Doppelbindungen, die im nichtumgesetzten Olefin noch vorhanden sind.

**Patentansprüche**

1. Verfahren zur Umsetzung von olefinisch ungesättigten Kohlenwasserstoffen, insbesondere von dimeren bis polymeren Isobutenderivaten, die ein mittleres Molekulargewicht mit einem Zahlenmittel $M_n$ von 100 bis 3000 aufweisen, mit Maleinsäureanhydrid im molaren Mengenverhältnis Maleinsäureanhydrid zu Olefin von 0,2 bis 3,0 in Gegenwart von 1 bis 5000 Gew.-ppm auf das Olefin, eines Nebenreaktionen verhindernden Zusatzstoffes bei Temperaturen zwischen 150 und 280°C unter Bildung der entsprechenden Bernsteinsäureanhydride, dadurch gekennzeichnet, dass als Zusatzstoffe Alkoxide des Titans, Zirkons, Vanadins oder Aluminiums verwendet werden.

2. Verfahren nach Anspruch 1, dadurch gekennzeichnet, dass $C_2$- bis $C_4$-Alkoxide eingesetzt werden.

3. Verwendung der nach Anspruch 1 hergestellten Bernsteinsäureanhydride mit mittleren Molekulargewichten $M_n$ von 200 bis 350 zur Herstellung von Korrosionsschutzmitteln.

4. Verwendung der nach Anspruch 1 hergestellten Bernsteinsäureanhydride mit mittleren Molekulargewichten $M_n$ von 250 bis 3000 zur Herstellung von Mineralöhilfsmitteln.

**Claims**

1. A process for the reaction of an olefinically unsaturated hydrocarbon, in particular a dimeric or higher polymeric isobutene derivative, which has a mean number average molecular weight $M_n$ of from 100 to 3000, with maleic anhydride in a molar ratio of maleic anhydride to olefin of from 0.2 to 3.0, in the presence of from 1 to 5000 ppm by weight, based on the olefin, of an additive which prevents side reactions, at from 150 to 280°C, with formation of the corresponding succinic anhydride, wherein the additive used is an alkoxide of titanium, of zirconium, of vanadium or of aluminum.

2. A process as claimed in claim 1, wherein a $C_2$-$C_4$-alkoxide is used.

3. The use of a succinic anhydride which has a mean number average molecular weight $M_n$ of from 200 to 350 and has been prepared according to claim 1, for the production of anticorrosive agents.

4. The use of a succinic anhydride which has a mean number average molecular weight $M_n$ of from 200 to 3000 and has been prepared according to claim 1, for the production of mineral oil additives.

**Revendications**

1. Procédé de réaction d'hydrocarbures à insaturation oléfinique, en particulier de dérivés d'isobutène dimères à polymères d'un poids moléculaire moyen en nombre $M_n$ de 100 à 3000, avec l'anhydride maléique dans un rapport molaire en poids de l'anhydride maléique à l'oléfine compris entre 0,2 et 3,0, à des températures comprises entre 150 et 280°C et en présence de 1 à 5000 ppm en poids par rapport à l'oléfine d'un additif inhibant des réactions secondaires, le produit réactionnel étant l'anhydride succinique correspondant, caractérisé en ce que les additifs sont choisis parmi les alcoxydes du titane, du zirconium, du vanadium et de l'aluminium.

2. Procédé suivant la revendication 1, caractérisé en ce que l'on emploie des alcoxydes en $C_2$ à $C_4$.

3. Utilisation des anhydrides succiniques préparés (par le procédé) selon la revendication 1, d'un poids moléculaire moyen en nombre $M_n$ de 200 à 350, pour la préparation d'inhibiteurs de la corrosion.

4. Utilisation des anhydrides succiniques préparés (par le procédé) selon la revendication 1, d'un poids moléculaire moyen en nombre $M_n$ de 250 à 3000, pour la préparation d'additifs pour huiles minérales.